Europäisches Patentamt

European Patent Office

Office européen des brevets

(19)

(11) **EP 0 959 793 B1**

(12) **EUROPÄISCHE PATENTSCHRIFT**

(45) Veröffentlichungstag und Bekanntmachung des
Hinweises auf die Patenterteilung:
**11.12.2002 Patentblatt 2002/50**

(51) Int Cl.⁷: $A61B\ 17/72$

(21) Anmeldenummer: **98907929.8**

(22) Anmeldetag: **07.01.1998**

(86) Internationale Anmeldenummer:
**PCT/EP98/00060**

(87) Internationale Veröffentlichungsnummer:
**WO 98/030163 (16.07.1998 Gazette 1998/28)**

(54) **DISTRAKTIONSVORRICHTUNG ZUM AUSEINANDERBEWEGEN ZWEIER TEILE EINES KNOCHENS**

DISTRACTION DEVICE FOR MOVING APART TWO BONE SECTIONS

DISPOSITIF DE DISTRACTION POUR ECARTER L'UNE DE L'AUTRE DEUX PARTIES D'UN OS

(84) Benannte Vertragsstaaten:
**AT CH DE ES FR GB IT LI**

(30) Priorität: **07.01.1997 DE 19700225**

(43) Veröffentlichungstag der Anmeldung:
**01.12.1999 Patentblatt 1999/48**

(73) Patentinhaber:
• **Wittenstein Motion Control GmbH
97999 Igersheim (DE)**
• **Betz, Augustin
78479 Reichenau (DE)**

(72) Erfinder:
• **BETZ, Augustin, Dr.
D-78479 Konstanz (DE)**
• **BUTSCH, Michael
D-88718 Daisendorf (DE)**

(74) Vertreter: **Manitz, Finsterwald & Partner
Postfach 31 02 20
80102 München (DE)**

(56) Entgegenhaltungen:
**DE-A- 3 921 972          DE-A- 19 624 295
US-A- 5 415 660**

**Beschreibung**

Die vorliegende Erfindung betrifft eine Distraktionsvorrichtung zum Auseinanderbewegen zweier Teile eines Knochens, insbesondere zur Knochenverlängerung oder zur Überbrückung einer Knochenlücke, mit einem in den Markraum eines Knochens einführbaren Marknagel, welcher zwei axial auseinanderfahrbare, jeweils an einem der beiden Knochenteile befestigbare Teile aufweist, mit einer eine Antriebswelle antreibenden Antriebseinheit und mit einer Vorrichtung zur Umsetzung der Rotationsbewegung der Antriebswelle in eine relative Axialbewegung der beiden Teile des Marknagels zueinander.

Die Verwendung eines Marknagels als Teil einer solchen Distraktionsvorrichtung ist aus der Druckschrift DE 39 21 972 C2 bekannt. Diese Vorrichtung dient insbesondere zur Verlängerung von Röhrenknochen oder Defektüberbrückung nach Trümmerbrüchen, einer Knochenentzündung oder nach Entfernen von Tumoren im Bereich langer Röhrenknochen. Diese bekannte Vorrichtung hat bereits erhebliche Vorteile gegenüber äußeren Distraktionsvorrichtungen, bei denen die Knochenteile durch die Haut nach außen mit einem verstellbaren Rahmen verbunden sind, da hierdurch eine ständige Infektionsgefahr durch Eindringen von Keimen sowie ein ungünstiger Kraftansatz gegeben ist.

Die Funktionsweise des bekannten Marknagels ist derart, daß durch Auseinanderbewegen der beiden Teile des Marknagels die beiden Teile des Knochens langsam auseinanderbewegt werden, wobei die sich dadurch bildende Lücke zwischen den beiden Knochenenden aufgrund des langsamen Vorschubes fortlaufend durch sich neu bildende Knochensubstanz überbrückt wird. Auf diese Weise können Knochen nicht nur verlängert werden, indem Knochen nach Aufbohren und Einführen des Marknagels in den Markraum durchtrennt und anschließend auseinandergezogen werden, sondern es können auch Knochenlücken überbrückt werden, indem ein von einem der Lücke benachbarten Knochenende abgetrenntes Knochenstück vom einen zum anderen Ende der Lücke bewegt wird. Entsprechend kann auch das Ende eines Knochenstumpfes, also ein Knochen mit fehlendem Knochenende, verlängert werden.

Wichtig bei einer Vorrichtung der eingangs genannten Art ist, daß die Auseinanderbewegung der Knochenteile langsam erfolgt, um ausreichend Zeit zur Bildung von Knochensubstanz und zur Anpassung des umliegenden Gewebes zu geben.

Der Erfindung liegt die Aufgabe zugrunde, eine Distraktionsvorrichtung der eingangs genannten Art anzugeben, die sich durch eine hohe Funktionssicherheit auszeichnet und eine möglichst geringe Baugröße mit zugleich hohem Wirkungsgrad der Antriebsvorrichtung ermöglicht.

Diese Aufgabe wird dadurch gelöst, daß die Antriebswelle Planetenrollen antreibt, die auf Umlaufbahnen gehalten sind, auf denen sie mit auf ihrem Außenumfang vorhandenen Antriebsrillen in korrespondierende Antriebsrillen eines die Planetenrollen umfassenden Hohlkörpers eingreifen, wobei zumindest die Antriebsrillen des Hohlkörpers oder der Planetenrollen als Gewinderillen ausgebildet sind, um den Hohlkörper bei einer Rotation der Antriebswelle relativ zu dieser axial zu verschieben.

Die erfindungsgemäße Distraktionsvorrichtung zeichnet sich durch einen hohen Wirkungsgrad ihrer Antriebsvorrichtung aus. Hierdurch wird einerseits eine hohe Funktionssicherheit gewährleistet und andererseits eine geringe Baugröße der Vorrichtung ermöglicht, da die Antriebseinrichtung trotz der erforderlichen hohen Kräfte verhältnismäßig klein ausgebildet werden kann. Die erfindungsgemäße Distraktionsvorrichtung ist daher besonders zur Implantation in den Körper des behandelten Patienten geeignet.

Zum Antrieb der Planetenrollen ist die Antriebswelle bevorzugt mit einer ritzelartigen Außenverzahnung versehen, welche in einer an jeder Planetenrolle neben den Antriebsrillen vorhandenen Außenverzahnung kämmt. Eine andere Möglichkeit besteht darin, die Antriebswelle drehfest mit einem in dem Hohlkörper rotierbaren Käfig zu verbinden, in welchem die Planetenrollen gelagert sind. Diese zweite Variante ist von der Ausgestaltung her einfacher, jedoch entfällt die Übersetzung der Zahnradstufe.

Nach einer weiteren Ausgestaltung der Erfindung umgibt der Hohlkörper die Antriebseinheit teleskopartig und bildet einen Teil des Marknagels. Dies führt zu einer geringen Baugröße der Distraktionsvorrichtung, die aufgrund der dadurch erzielbaren geringen Länge des Marknagels auch bei kleinen und kurzen Knochen einsetzbar ist.

Nach weiteren Ausgestaltungen der Erfindung kann die Antriebseinheit gegenüber einem Knochen fixierbar und der Hohlkörper ausfahrbar ausgebildet sein, oder aber es kann der Hohlkörper an einem Knochen fixierbar und die Antriebseinheit mit den Planetenrollen und einem koaxial zum Hohlkörper gelagerten, axial belastbaren Stift ausfahrbar sein. Die zweite Variante hat den Vorteil, daß der im Durchmesser größere Hohlkörper im proximalen Ende eines Röhrenknochens angeordnet werden kann, während der im Durchmesser kleinere Stift im distalen Ende angeordnet werden kann, wo weniger Platz vorhanden ist. Die Belastung durch Einbringung des Marknagels ist dadurch verringert.

Durch Vorsehen eines Kardangelenkes zwischen Anriebsmotor und Hohlkörper kann der Marknagel vorteilhafterweise auch gekrümmt ausgebildet werden. Insbesondere kann auch ein einen Teil des Marknagels bildender Hohlkörper mit Krümmungen versehen sein. Durch Vorsehen einer Krümmung des Marknagels kann dieser an den Markraum eines Knochens anatomisch angepaßt werden. Die Beeinträchtigung des Knochens durch die Distraktionsvorrichtung wird hierdurch verringert. Zusammen mit der durch die Erfindung ermöglichten kleinen Baugröße des Marknagels kann dieser so ausgebildet sein, daß er ohne Aufbohren der Knochensubstanz in den Markraum eines Marknagels einführbar ist. Eine Schwächung des Knochens wird hierdurch vermieden, zumindest jedoch verringert.

Nach einer weiteren Ausgestaltung der Erfindung treibt der Hohlkörper den Kolben einer Hydraulikpumpe an, durch welche der Marknagel antreibbar ist. Die Antriebsvorrichtung kann auf diese Weise getrennt von dem Marknagel untergebracht und insbesondere im Körper des Patienten implantiert werden. Auch diese Ausgestaltung ermöglicht eine Ausgestaltung des Marknagels mit Krümmungen. Vorteilhaft ist es dabei, den Marknagel als Kolben-Zylindereinheit auszubilden. Ein Teil des Marknagels bildet dann den Kolben, der durch die Hydraulikpumpe antreibbar ist.

Nach einer weiteren Ausgestaltung der Erfindung umfaßt die Antriebseinheit einen Elektromotor, der bevorzugt induktiv, insbesondere durch Hochfrequenzeinkopplung mit Energie versorgbar ist. Ebenso ist es aber auch möglich, den Elektromotor durch einen implantierbaren Energiespeicher anzutreiben. In beiden Fällen ist gewährleistet, daß keine Infektionsgefahr durch nach außen geführte Teile, beispielsweise Anschlußdrähte, der Distraktionsvorrichtung besteht.

Nach einer weiteren Ausgestaltung der Erfindung ist eine in den Körper des Patienten implantierbare Kraft- und/oder Wegmeßeinrichtung für die Bewegung des Marknagels vorgesehen. Durch diese Meßeinrichtung kann der Knochenaufbauprozeß überwacht und protokolliert werden, ohne daß der Patient durchleuchtet werden muß und somit ohne die Gefahr einer dadurch hervorgerufenen Belastung des Patienten.

Als Kraftmeßeinrichtung ist nach einer weiteren Ausgestaltung der Erfindung bevorzugt ein Dehnungsmeßstreifen vorgesehen, während die Wegmeßeinrichtung beispielsweise potentiometrisch arbeiten kann. Eine andere vorteilhafte Möglichkeit besteht darin, einen an den Motor adaptierten Encoder oder Hallsensor zu verwenden. Die Übertragung der Meßsignale nach außen kann beispielsweise mittels eines Telemetriesystems erfolgen.

Die für das Verhältnis zwischen Drehzahl der Antriebswelle und Axialweg des Hohlkörpers maßgebenden Parameter der sich bewegenden Teile der erfindungsgemäßen Vorrichtung lassen sich in einer nach dem Verschiebeweg aufgelösten Gleichung wie folgt zusammenfassen:

$$S_{ax} = U_a \cdot (Ps \cdot \frac{1}{d_{wa} + d_{wr}} - Pr \cdot \frac{d_{gs}}{d_{gr} \cdot d_{gr}}) \cdot \frac{d_{wa} \cdot d_{gr}}{d_{gr} + d_{wr}}$$

In dieser Gleichung bedeuten

$S_{ax} =$ Axialweg des Hohlkörpers
$U_a =$ Anzahl der Umdrehungen der Antriebswelle
$Ps=$ Steigung der Rillen des Hohlkörpers

$Pr=$ Steigung der Rillen der Planetenrollen
$d_{gs} =$ Durchmesser der Rillen des Hohlkörpers
$d_{gr} =$ Durchmesser der Rillen der Planetenrollen
$d_{wa} =$ Wälzkreisdurchmesser der Verzahnung des Antriebswellen-Ritzels
$d_{wr} =$ Wälzkreisdurchmesser der Verzahnung der Planetenrollen.

Unter Verwendung der oben zitierten Gleichung wird nachfolgend ein konkretes Beispiel angegeben.

Ua = 1 Umdrehung
Ps= 1 mm
Pr= 0
$d_{gs}$ = 10 mm
$d_{gr} = d_{wr}$ = 3 mm
$d_{wa}$ = 4 mm

Mit den vorstehenden Werten ergibt sich für eine Umdrehung der Antriebswelle ein axialer Verschiebeweg des Hohlkörpers von $S_{ax}$ = 0,286 mm.

Im Falle der direkten Verbindung von Antriebswelle und Käfig ist für Pr = 0:

$$S_{ax} = U_a \cdot Ps.$$

Die Planetenrollen können im Bereich ihrer Antriebsrillen ohne direkten form- und/oder kraftschlüssigen Kontakt mit der Antriebswelle sein. Sie können insbesondere in einem frei rotierbaren Käfig gelagert sein, welcher seinerseits an dem Gehäuse der Antriebswelle axial fixiert wälzgelagert ist. Die Reibung der Vorrichtung zur Umwandlung einer Dreh- in eine Axialbewegung wird dadurch im wesentlichen günstigerweise auf Rollreibung reduziert.

Der Käfig kann dazu dienen, die Planetenrollen zu führen und die von den Planetenrollen ausgehenden Axialkräfte aufzunehmen. Die Axialkräfte der Planetenrollen können aber auch direkt von der Antriebswelle aufgenommen werden. In diesem Fall ist die Antriebswelle axial fixiert an dem Antriebsgehäuse zu lagern.

Zur Übertragung der axialen Kräfte von den Planetenrollen auf die Antriebswelle können die Planetenrollen zusätzlich zu ihren Antriebsrillen mit Lagerrillen versehen sein, die in entsprechende Lagerrillen der Antriebswelle axial kraftabstützend eingreifen.

Die in die Lagerrillen der Planetenrollen eingreifenden Gegenrillen bzw. Gegenprofile müssen nicht Bestandteil der Antriebswelle sein. Sie müssen lediglich drehbar und gegenüber dem Antriebsgehäuse axial fixiert sein.

Grundsätzlich ist es auch möglich, daß die Antriebsrillen der Planetenrollen gleichzeitig als deren Lagerrillen zur axialen Abstützung an einem rotierenden radial innerhalb der Umlaufbahn der Planetenrollen liegenden Lagerkörper dienen.

Der Lagerkörper kann auch in diesem Fall fest mit der Antriebswelle verbunden sein, wobei dann allerdings die Antriebsrillen der Planetenrollen sowie die Lagerrillen des Lagerkörpers jeweils steigungslos ausgebildet sein sollten. Ferner sollten bei einer solchen Ausführung die Werte der mittleren Durchmesser der Rillen des Lagerkörpers und des Wälzkreises der Verzahnung zwischen dem Antriebs-Ritzel der Antriebswelle und den Planetenrollen übereinstimmen.

Die Innenrillierung des Hohlkörpers kann insbesondere ein mehrgängiges Gewinde sein. Bei bestimmten Rillen-Kombinationen der drehbar ineinandergreifenden Teile der Antriebsvorrichtung ist ein mehrgängiges Gewinde insbesondere mit Bezug auf die Rillierung des Hohlkörpers notwendig.

Die erfindungsgemäße Vorrichtung besitzt den Vorteil, daß der Hohlkörper in der Form eines Rohres über eine Strecke ausgefahren werden kann, auf der zuvor kein Führungskörper vorhanden sein muß. Durch die Ausbildung als Rohr besitzt der Hohlkörper eine hohe Knickfestigkeit. Darüber hinaus sind die innerhalb des Rohres liegenden Antriebselemente einfach und sicher abdichtbar.

Ausführungsbeispiele der Erfindung sind in der Zeichnung dargestellt und werden nachfolgend beschrieben. Es zeigen, jeweils in schematischer Darstellung,

Fig. 1 eine erfindungsgemäße Distraktionsvorrichtung in teilweise geschnittener Darstellung mit zusammengefahrenem Marknagel,

Fig. 2 einen Querschnitt gemäß Linie SI-SI in Figur 1,

Fig. 3 den Marknagel einer zweiten Variante der erfindungsgemäßen Distraktionsvorrichtung in teilweise geschnittener Darstellung,

Fig. 4 einen Querschnitt gemäß Linie SI-SI in Fig. 3,

Fig. 5 einen Querschnitt gemäß Linie SII-SII in Fig. 3,

Fig. 6 einen Ausschnitt aus einer dritten Variante der erfindungsgemäßen Distraktionsvorrichtung,

Fig. 7 einen Ausschnitt aus einer vierten Variante der erfindungsgemäßen Distraktionsvorrichtung,

Fig. 8 einen Ausschnitt aus einer fünften Variante der erfindungsgemäßen Distraktionsvorrichtung,

Fig. 9 einen Ausschnitt aus einer sechsten Variante der erfindungsgemäßen Distraktionsvorrichtung,

Fig. 10 einen Querschnitt gemäß Linie SI-SI in Fig. 9, und

Fig. 11 einen Ausschnitt aus einer siebten Variante der erfindungsgemäßen Distraktionsvorrichtung.

Die erfindungsgemäße Distraktionsvorrichtung umfaßt einen in den Markraum eines Knochens einführbaren Marknagel 1 welcher zwei axial auseinanderfahrbare, jeweils an einem der beiden Knochenteile befestigbare Teile 2, 3 aufweist, sowie eine Antriebseinheit 4 zum Auseinanderfahren der beiden Teile 2, 3 des Marknagels 1. Die Antriebseinheit 4 ist im Inneren des Marknagels 1 angeordnet und induktiv über eine im Körper des Patienten implantierbare Spule 5 und elektrische Leitungen 6 von außen mit Energie versorgbar. Ebenso wäre eine Energieversorgung über eine implantierte Batterie möglich.

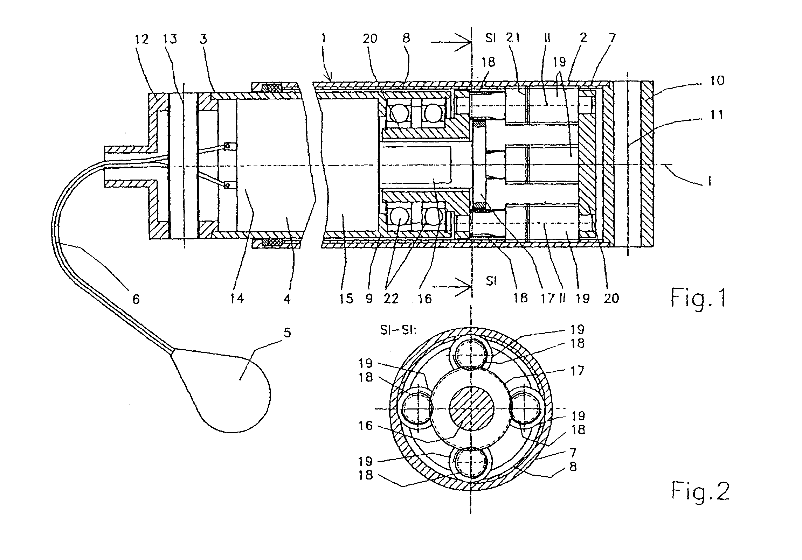

Wie man in Fig. 1 erkennt, wird der erste Teil 2 des Marknagels 1 durch einen rohrförmigen Hohlkörper 7 gebildet, welcher auf seiner Innenumfangsfläche mit einer Rillierung 8 versehen ist. In das eine Ende des Rohres 7 eingeschoben ist der zweite Teil 3 des Marknagels 1, der ein ebenfalls rohrförmiges Gehäuse 9 umfaßt, während in die andere Seite des Rohres 7 ein Endstück 10 eingesetzt ist. In dem Endstück 10 ist eine Durchgangsöffnung 11 vorhanden, durch welche hier nicht dargestellte Befestigungsmittel, insbesondere eine Schraube, hindurchführbar sind, mit denen der erste Teil 2 des Marknagels 1 an einem Knochen oder Knochenstück befestigbar ist.

In das dem Endstück 10 abgewandte Ende des Gehäuses 9 ist ebenfalls ein Endstück 12 eingesetzt, das ebenfalls mit einer Durchgangsöffnung 13 versehen ist, in welche Befestigungsmittel zur Befestigung des zweiten Teiles 3 des Marknagels 1 an einem Knochen oder Knochenstück, insbesondere eine Befestigungsschraube, einführbar sind. Die über das Endstück 12 zugeführten elektrischen Anschlußdrähte 6 sind an einen im Inneren des Gehäuses 9 vorhandenen Elektromotor 14 angeschlossen. Der Elektromotor 14 treibt über ein ebenfalls in dem Gehäuse 9 vorhandenes Getriebe 15 eine Antriebswelle 16 an, deren Längsachse mit der Längsachse I des Marknagels 1 zusammenfällt.

Die Antriebswelle 16 ist mit einer ritzelartigen Verzahnung 17 versehen, die in korrespondierenden Außenverzahnungen 18 von vier Planetenrollen 19 kämmt, die die Ritzelverzahnung 17 gleichmäßig umgeben und mittels eines Käfigs 20 fixiert sind, wobei sich die Längsachsen II der Planetenrollen 19 parallel zur Längsachse I des Marknagels 1 erstrecken.

Die Planetenrollen 19 sind zusätzlich mit einer Rillierung 21 ihrer Außenseite versehen, die mit der Rillierung 8 auf der Innenumfangsseite des Hohlkörpers 7 in Eingriff steht. Die Rillierung 8 des Hohlkörpers 7 ist bei dieser Ausgestaltung als Gewinderille ausgebildet, weist also eine Steigung in Richtung der Längsachse I auf. Auf diese Weise führt eine durch die ritzelartige Verzahnung 17 auf die Planetenrollen 19 übertragene Rotation der Antriebswelle 16 zu einer entsprechenden Axialbewegung des Hohlkörpers 7, während die Planetenrollen 19 gegenüber der Antriebswelle 16 durch den Käfig 20 axial fixiert sind. Der Käfig 20 kann zweckmäßigerweise mit Wälzlagern 22 zum Gehäuse 9 abgelagert werden.

Die Funktionsweise des erfindungsgemäßen Marknagels ist derart, daß der über die Induktionsspule 5 von außerhalb des Körpers des Patienten mit Energie versorgte Elektromotor 14 über das Getriebe 15 die Antriebswelle 16 in Rotation versetzt, die ihrerseits über die Ritzelverzahnung 17 und die Außenverzahnung 18 die Planetenrollen 19 in Rotation versetzt. Durch die Rotation der axial gegenüber der Antriebswelle 16 fixierten Planetenrollen 19 wird der Hohlkörper 7 aufgrund des Eingriffs seiner Rillierung 8 mit der Rillierung 21 der Planetenrollen 19 ausgefahren. Ein an dem Hohlkörper 7 über die Durchgangsöffnung 11 befestigtes Knochenstück wird dabei von einem zweiten Knochenstück, welches über die Durchgangsöffnung 13 mit dem Gehäuse 9 verbunden ist, wegbewegt. Die Bewegung kann dabei aufgrund des hohen Wirkungsgrades der erfindungsgemäßen Antriebseinrichtung sehr langsam und mit sehr hoher Kraft erfolgen. Der hohe Wirkungsgrad ergibt sich dabei vor allem aus der Tatsache, daß zwischen den bewegten Elementen des Marknagels im wesentlichen Rollreibung und keine Gleitreibung auftritt. Zwischen den beiden auseinanderbewegten Knochenstücken bildet sich neue Knochensubstanz, welche die Knochenlücke schließt.

Ein Knochen kann auf diese Weise also verlängert werden. Ebenso ist es möglich, eine vorhandene Lücke in einem Knochen zu überbrücken, indem ein von einem Ende des Knochens abgetrenntes Knochenstück zum anderen Ende der Lücke bewegt wird. In diesem Fall ist es auch möglich, die erfindungsgemäße Vorrichtung so einzusetzen, daß der Hohlkörper 7 zur Bewegung des Knochenstückes nicht aus- sondern eingefahren wird.

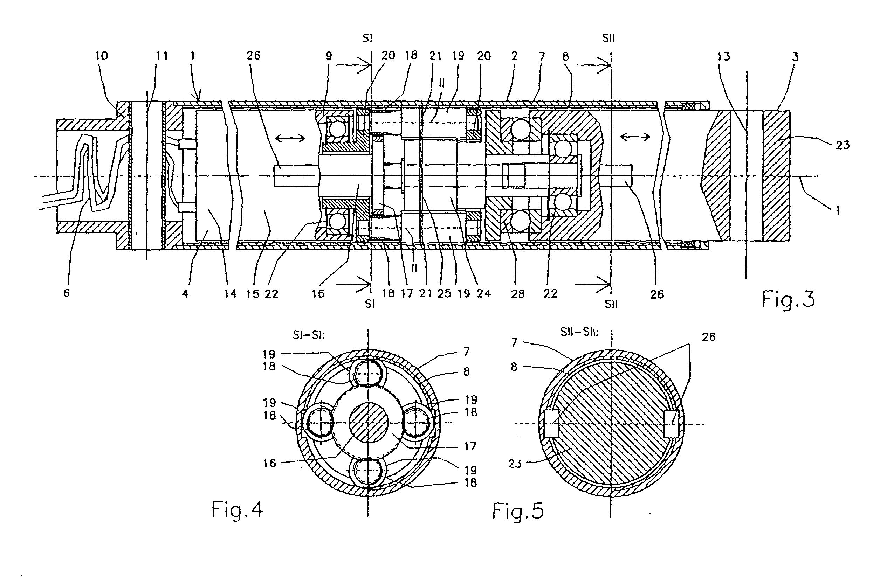

Bei den in den Figuren 3 bis 8 und 11 dargestellten Ausführungsbeispielen werden die auf die Planetenrollen 19 wirkenden Axialkräfte nicht vom Käfig 20 aufgenommen. Statt dessen ist innerhalb der Planetenrollen 19 ein Lagerkörper 24 angeordnet, der eine Rillierung 25 aufweist, die in die Rillierung 21 bzw. 27 der Planetenrollen 19 formschlüssig eingreift.

Die in Fig. 3 dargestellte Variante unterscheidet sich von der Variante von Fig. 1 zusätzlich dadurch, daß hier der Hohlkörper 7 am Knochen fixierbar ist, während bei'Betätigung der Vorrichtung die Antriebseinheit 4 mit Antriebswelle 16, Planetenrollen 19, Lagerkörper 24 und ein axial belastbarer Stift 23 axial ausfahren, der über ein Axiallager 28 mit dem Lagerkörper 24 verbunden ist. Zwischen den beiden Teilen 2 und 3 des Marknagels 1 vorhandene, in dessen Längsrichtung erstreckte Paßstücke 26 gewährleisten dabei eine drehfeste Fixierung der beiden Teile 2 und 3 gegeneinander. Über die Durchgangsöffnung 13 ist der Stift 23 an einem Knochenstück befestigbar, welches beim Ausfahren verschoben werden soll.

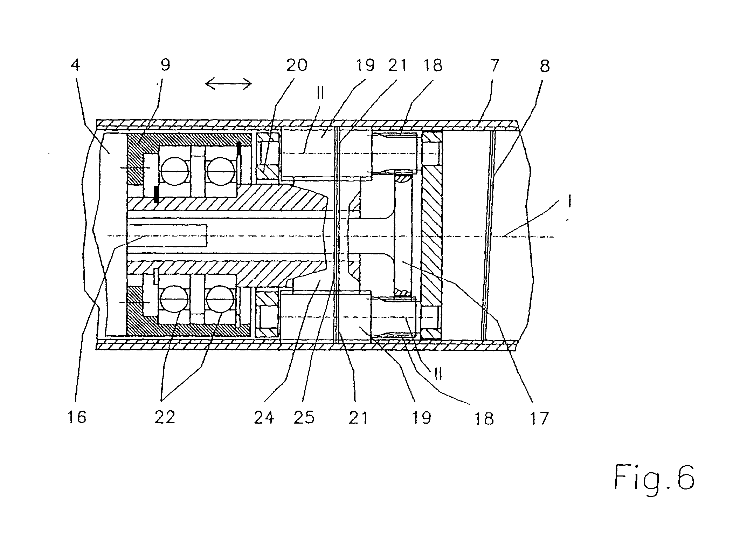

Bei der in Fig. 6 dargestellten Variante durchsetzt die Antriebswelle 16 den Lagerkörper 24. Die Ritzelverzahnung 17 ist auf der der Antriebseinheit 4 abgewandten Seite der Antriebswelle 16 angeordnet. Der die Axialkräfte aufnehmende Lagerkörper 24 ist über Wälzlager 22 zum Gehäuse 9 hin gelagert.

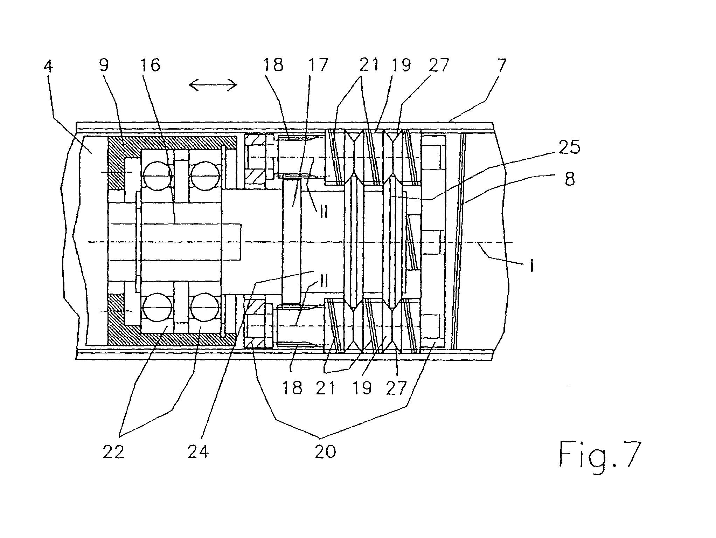

Die in Fig. 7 gezeigte Ausgestaltung unterscheidet sich von den in Fig. 3 und 6 gezeigten Ausgestaltungen dadurch, daß die Planetenrollen 19 zwei unterschiedliche Rillierungen 21 und 27 aufweisen, von denen die Rillierung 21 mit der Rillierung 8 des Hohlkörpers 7 in Eingriff steht, während die Rillierung 27 in die Rillierung 25 des Lagerkörpers 24 eingreift. Auf diese Weise kann auch die Rillierung 21 der Planetenrollen 19 mit einer Steigung ausgebildet sein, während die Rillierung 27 wie die Rillierung 25 des Lagerkörpers 24 keine Steigung aufweist, um die Planetenrollen 19 gegenüber der Antriebswelle 16 axial zu fixieren.

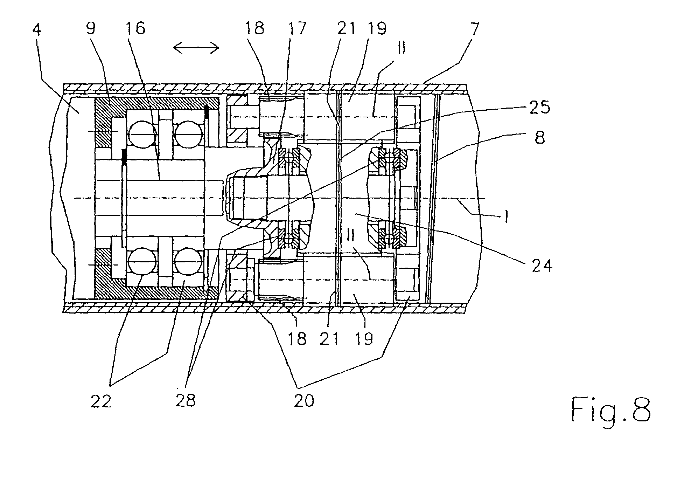

Die Ausgestaltung gemäß Fig. 8 unterscheidet sich von den Ausgestaltungen gemäß Fig. 3, 6 und 7 dadurch, daß der Lagerkörper 24 gegenüber der Antriebswelle 16 zwar axial fixiert, jedoch frei drehbar gelagert ist. Hierfür sind zwei als Kugelrollenlager ausgebildete Axiallager 28 vorgesehen, von denen jeweils eines an jeder der beiden Stirnseiten des Lagerkörpers 24 vorgesehen ist. Aufgrund dieser Ausgestaltung kann der Wälzkreisdurchmesser der Ritzelverzahnung 17 der Antriebswelle 16 unabhängig vom mittleren Durchmesser der Lagerrillen 25 des Lagerkörpers 24 gewählt werden.

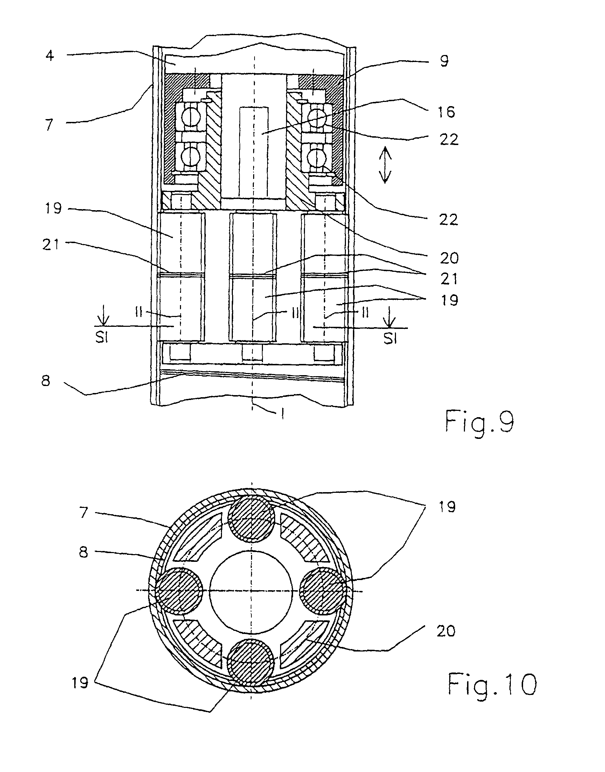

Bei der in Fig. 9 dargestellten Variante ist die Antriebswelle 16 direkt mit dem Käfig 20 der Planetenrollen 19 verbunden, die daher nur mit einer Rillierung 21, jedoch keiner Außenverzahnung versehen sind. Die Planetenrollen werden somit über den Käfig längs ihrer Umlaufbahnen bewegt. Die Axialkräfte werden vom Käfig 20 über die Wälzlager 22 auf das Gehäuse 9 geleitet.

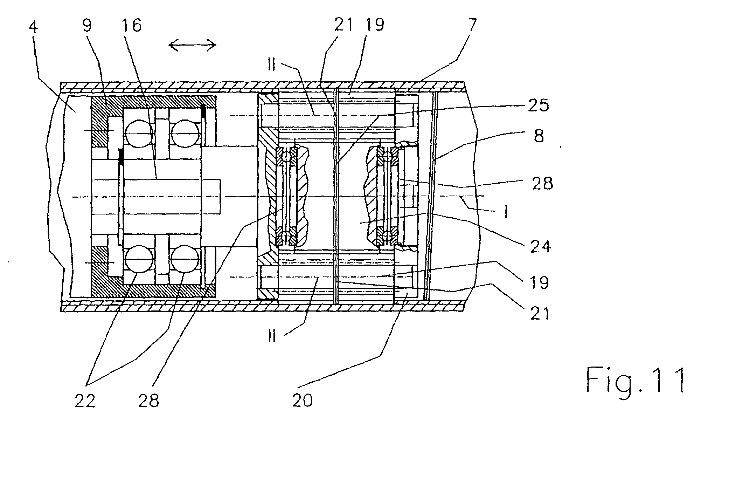

Fig. 11 zeigt eine Ausgestaltung, bei der wie bei der Variante nach Fig. 9 der Käfig 20 der Planetenrollen 19 direkt angetrieben ist. Die Abstützung der Axialkräfte erfolgt über einen Lagerkörper 24, dessen Rillierung 25 formschlüssig in Eingriff mit der Rillierung 21 der Planetenrollen 19 steht.

Der Lagerkörper 24 wird über Axiallager 28 an der Antriebswelle 16 abgestützt, die wiederum über die Wälzlager 22 im Gehäuse 9 gelagert ist.

Die Ritzelverzahnung 17 bzw. der Käfig 20 können in allen Fällen auch über eine Kupplung kardanisch angetrieben werden, so daß zwischen der Antriebswelle 16 und der Achse des Hohlkörpers 7 ein Schwenkwinkel realisiert werden kann.

**Patentansprüche**

1. Distraktionsvorrichtung zum Auseinanderbewegen zweier Teile eines Knochens, insbesondere zur Knochenverlängerung oder zur Überbrückung einer Knochenlücke, mit einem in den Markraum eines Knochens einführbaren Marknagel (1), welcher zwei axial auseinanderfahrbare, jeweils an einem der beiden Knochenteile befestigbare Teile (2, 3) aufweist, mit einer eine Antriebswelle (16) antreibenden Antriebseinheit (4) und mit einer Vorrichtung zur Umsetzung der Rotationsbewegung der Antriebswelle (16) in eine relative Axialbewegung der beiden Teile (2, 3) des Marknagels (1) zueinander,
   **dadurch gekennzeichnet,**
   **daß** die Antriebswelle (16) Planetenrollen (19) antreibt, die auf Umlaufbahnen gehalten sind, auf denen sie mit auf ihrem Außenumfang vorhandenen Antriebsrillen (21) in korrespondierende Antriebsrillen (8) eines die Planetenrollen (19) umfassenden Hohlkörpers (7) eingreifen, wobei zumindest die Antriebsrillen (8) des Hohlkörpers (7) oder der Planetenrollen (19) als Gewinderillen ausgebildet sind, um den Hohlkörper (7) bei einer Rotation der Antriebswelle (16) relativ zu dieser axial zu verschieben.

2. Distraktionsvorrichtung nach Anspruch 1,
   **dadurch gekennzeichnet,**
   **daß** die Antriebswelle (16) zum Antrieb der Planetenrollen (19) mit einer ritzelartigen Außenverzahnung (17) versehen ist, welche in einer an jeder Planetenrolle (19) neben den Antriebsrillen (21) vorhandenen Außenverzahnung (18) kämmt.

3. Distraktionsvorrichtung nach Anspruch 1,
   **dadurch gekennzeichnet,**
   **daß** die Antriebswelle (16) zum Antrieb der Planetenrollen (19) drehfest mit einem in dem Hohlkörper (7) rotierbaren Käfig (20) verbunden ist, in welchem die Planetenrollen (19) gelagert sind.

4. Distraktionsvorrichtung nach einem der vorhergehenden Ansprüche,
   **dadurch gekennzeichnet,**
   **daß** der Hohlkörper (7) die Antriebseinheit (4) teleskopartig umgibt und einen Teil (2) des Marknagels bildet.

5. Distraktionsvorrichtung nach Anspruch 4,
   **dadurch gekennzeichnet,**
   **daß** die Antriebseinheit (4) gegenüber einem Knochen fixierbar und der Hohlkörper (7) ausfahrbar ausgebildet ist.

6. Distraktionsvorrichtung nach Anspruch 4,
   **dadurch gekennzeichnet,**
   **daß** der Hohlkörper (7) an einem Knochen fixierbar und die Antriebseinheit (4) mit den Planetenrollen (19) und einem koaxial zum Hohlkörper (7) gelagerten axial belastbaren Stift (23) ausfahrbar ist.

7. Distraktionsvorrichtung nach einem der vorhergehenden Ansprüche,
   **dadurch gekennzeichnet,**
   **daß** zwischen Antriebseinheit (4) und Hohlkörper (7) ein Kardangelenk vorhanden ist.

8. Distraktionsvorrichtung nach einem der Ansprüche 1 bis 3,
   **dadurch gekennzeichnet,**
   **daß** der Hohlkörper (7) den Kolben einer Hydraulikpumpe antreibt, durch welche der Marknagel antreibbar ist.

9. Distraktionsvorrichtung nach Anspruch 8,
   **dadurch gekennzeichnet,**
   **daß** der Antrieb und die Hydraulikpumpe im Körper des Patienten implantierbar sind.

10. Distraktionsvorrichtung nach einem der vorhergehenden Ansprüche,
    **dadurch gekennzeichnet,**
    **daß** die Antriebseinheit (4) einen Elektromotor (14) umfaßt.

11. Distraktionsvorrichtung nach Anspruch 10,
    **dadurch gekennzeichnet,**
    **daß** der Elektromotor (14) induktiv, insbesondere durch Hochfrequenzeinkopplung, mit Energie versorgbar ist.

12. Distraktionsvorrichtung nach Anspruch 10,
    **dadurch gekennzeichnet,**
    **daß** der Elektromotor (14) durch einen implantierbaren Energiespeicher antreibbar ist.

13. Distraktionsvorrichtung nach einem der vorhergehenden Ansprüche,
    **dadurch gekennzeichnet,**
    **daß** eine in den Körper des Patienten implantierbare Kraftund/oder Wegmeßeinrichtung für die Bewegung des Marknagels (1) vorgesehen ist.

14. Distraktionsvorrichtung nach Anspruch 13,
    **dadurch gekennzeichnet,**
    **daß** die Kraftmeßeinrichtung mit einem Dehnungsmeßstreifen arbeitet.

15. Distraktionsvorrichtung nach Anspruch 13 oder 14,
    **dadurch gekennzeichnet,**
    **daß** die Wegmeßeinrichtung potentiometrisch arbeitet.

**16.** Distraktionsvorrichtung nach Anspruch 13 oder 14, **dadurch gekennzeichnet,** **daß** als Wegmeßeinrichtung ein an den Motor adaptierter Encoder dient.

**17.** Distraktionsvorrichtung nach Anspruch 13 oder 14, **dadurch gekennzeichnet,** **daß** ein die Anzahl der Umdrehungen der Antriebswelle (16) registrierender Hallsensor vorgesehen ist.

**18.** Distraktionsvorrichtung nach einem der Ansprüche 13 bis 17, **dadurch gekennzeichnet,** **daß** eine Telemetrieanlage zur berührungslosen Übertragung der Meßsignale aus dem Körper des Patienten vorgesehen ist.

**19.** Distraktionsvorrichtung nach einem der vorhergehenden Ansprüche, **dadurch gekennzeichnet,** **daß** die Planetenrollen (19) im Bereich ihrer Antriebsrillen (21) keinen direkten form- und/oder kraftschlüssigen Kontakt gegenüber der Antriebswelle (16) besitzen.

**20.** Distraktionsvorrichtung nach einem der vorhergehenden Ansprüche, **dadurch gekennzeichnet,** **daß** die Planetenrollen (19) in einem rotierbaren Käfig (20) gelagert sind, welcher an dem Gehäuse (9) der Antriebswelle (16) axial fixiert wälzgelagert ist.

**21.** Distraktionsvorrichtung nach einem der Ansprüche 1 bis 18, **dadurch gekennzeichnet,** **daß** in den Planetenrollen (19) zusätzliche Lagerrillen (27) vorgesehen sind, mit denen die Planetenrollen (19) in entsprechend axial unbeweglichen Gegenlagerrillen (25) axial fixiert sind.

**22.** Distraktionsvorrichtung nach Anspruch 21, **dadurch gekennzeichnet,** **daß** die Lagerrillen (27) der Planetenrollen (19) in axial unverschiebbare Gegenlagerrillen (25) eines in der Achse (I) der Antriebswelle (16) rotierenden Lagerkörpers (24) eingreifen.

**23.** Distraktionsvorrichtung nach Anspruch 22, **dadurch gekennzeichnet,** **daß** der Lagerkörper (24) fest mit der Antriebswelle (16) verbunden ist und daß die Lagerrillen (27, 25) der Planetenrollen (19) und des Lagerkörpers (24) jeweils steigungslos sind.

**24.** Distraktionsvorrichtung nach Anspruch 23, **dadurch gekennzeichnet,**

**daß** die Werte des mittleren Durchmessers der Lagerrillen (25) des Lagerkörpers (24) und des Wälzkreisdurchmessers der Verzahnung zwischen dem Antriebsritzel (17) und den Planetenrollen (19) übereinstimmen.

**25.** Distraktionsvorrichtung nach Anspruch 22, **dadurch gekennzeichnet,** **daß** der Lagerkörper (24) axial fixiert, aber drehbar an der Antriebswelle (16) gelagert ist.

**Claims**

**1.** Traction apparatus for moving apart two parts of a bone, in particular for bone extension or to bridge a bone gap comprising a medullary pin (1) which can be introduced into the medullary space of a bone and which has two parts (2, 3) respectively securable to one of the two bone parts and moveable axially apart, a drive unit (4) which drives a drive shaft (16) and a device for converting the rotational movement of the drive shaft (16) into a relative axial movement of the two parts (2, 3) of the medullary pin (1), **characterized in that** the drive shaft (16) drives planetary rollers (19) which are held on orbits on which drive grooves (21) on their outer circumference engage in corresponding drive grooves (8) of a hollow body (7) surrounding the planetary rollers (19), with at least the drive grooves (8) of the hollow body (7) or of the planetary rollers (19) being formed as thread grooves in order, on a rotation of the drive shaft (16), to displace the hollow body (7) axially relative to the latter.

**2.** Traction apparatus in accordance with claim 1, **characterized in that** the drive shaft (16) is provided for the driving of the planetary rollers (19) with a pinion-like outer toothing (17) which meshes in an outer toothing (18) present at each planetary roller (19) alongside the drive grooves (21).

**3.** Traction apparatus in accordance with claim 1, **characterized in that** the drive shaft (16) is rotationally fixedly connected to a cage (20) rotatable in a hollow body (7) in which the planetary rollers (19) are journalled for the driving of the planetary rollers (19).

**4.** Traction apparatus in accordance with any one of the preceding claims, **characterized in that** the hollow body (7) telescopically surrounds the drive unit (4) and forms a part (2) of the medullary pin.

**5.** Traction apparatus in accordance with claim 4,
**characterized in that**
the drive unit (4) can be fixed relative to a bone and the hollow body (7) is extensibly designed.

**6.** Traction apparatus in accordance with claim 4,
**characterized in that**
the hollow body (7) can be fixed on one bone and the drive unit (4) with the planetary rollers (19) and an axially loadable pin (23) mounted coaxially to the hollow body (7) is extensible.

**7.** Traction apparatus in accordance with any one of the preceding claims,
**characterized in that**
a cardan joint is present between drive unit (4) and hollow body (7).

**8.** Traction apparatus in accordance with any one of the claims 1 to 3,
**characterized in that**
the hollow body (7) drives the piston of a hydraulic pump by which the medullary pin can be driven.

**9.** Traction apparatus in accordance with claim 8,
**characterized in that**
the drive and the hydraulic pump can be implanted in the body of the patient.

**10.** Traction apparatus in accordance with any one of the preceding claims,
**characterized in that**
the drive unit (4) includes an electric motor (14).

**11.** Traction apparatus in accordance with claim 10,
**characterized in that**
the electric motor (14) can be supplied with energy inductively, in particular by coupling in high frequency energy.

**12.** Traction apparatus in accordance with claim 10,
**characterized in that**
the electric motor (14) is drivable by an implantable energy store.

**13.** Traction apparatus in accordance with any one of the preceding claims,
**characterized in that**
a force and/or path measuring device implantable into the body of the patient is provided for the movement of the medullary pin (1).

**14.** Traction apparatus in accordance with claim 13,
**characterized in that**
the power measuring device operates with a strain gauge.

**15.** Traction apparatus in accordance with claim 13 or claim 14,
**characterized in that**
the path measuring device operates potentiometrically.

**16.** Traction apparatus in accordance with claim 13 or claim 14,
**characterized in that**
an encoder adapted to the motor serves as the path measuring device.

**17.** Traction apparatus in accordance with claim 13 or claim 14,
**characterized in that**
a Hall sensor is provided which registers the number of rotations of the drive shaft (16).

**18.** Traction apparatus in accordance with any one of the claims 13 to 17,
**characterized in that**
a telemetry system is provided for the contactless transmission of the measurement signals from the body of the patient.

**19.** Traction apparatus in accordance with any one of the preceding claims,
**characterized in that**
the planetary rollers (19) have, in the region of their drive grooves (21), no direct shape matched and/or force locked contact relative to the drive shaft (16).

**20.** Traction apparatus in accordance with any one of the preceding claims,
**characterized in that**
the planetary rollers (19) are journalled in a rotatable cage (20) which is axially fixedly rollingly journalled on the housing (9) of the drive shaft (16).

**21.** Traction apparatus in accordance with any one of the claims 1 to 18,
**characterized in that**
additional bearing grooves (27) are provided in the planetary rollers (19) with which the planetary rollers (19) are axially fixed in corresponding axially immovable counter-bearing grooves (25).

**22.** Traction apparatus in accordance with claim 21,
**characterized in that**
the bearing grooves (27) of the planetary rollers (19) engage into axially non-displaceable counter-bearing grooves (25) of a bearing body (24) rotating in the axis (I) of the drive shaft (16).

**23.** Traction apparatus in accordance with claim 22,
**characterized in that**
the bearing body (24) is fixedly connected to the drive shaft (16) and **in that** the bearing grooves (27, 25) of the planetary rollers (19) and of the bearing body (24) are each pitch-free.

24. Traction apparatus in accordance with claim 23, **characterized in that**
the values of the average diameter of the bearing grooves (25) of the bearing body (24) and of the rolling circle diameter of the toothing between the drive pinion (17) and the planetary rollers (19) correspond.

25. Traction apparatus in accordance with claim 22, **characterized in that**
the bearing body (24) is axially fixed but rotatably mounted on the drive shaft (16).

**Revendications**

1. Dispositif de distraction destiné à l'écartement l'une de l'autre de deux parties d'un os, notamment à l'élongation d'un os ou au pontage d'un interstice d'os, comportant une broche à moelle (1) pouvant être introduite dans le canal médullaire d'un os, qui est munie de deux parties (2, 3) pouvant être écartées axialement l'une de l'autre et fixées respectivement sur l'une des deux parties d'un os, une unité d'entraînement (4) entraînant un arbre d'entraînement (16), et un dispositif destiné à la conversion du mouvement de rotation de l'arbre d'entraînement (16) en un mouvement axial des deux parties (2, 3) de la broche à moelle (1) l'une par rapport à l'autre, **caractérisé en ce que**
l'arbre d'entraînement (16) entraîne des rouleaux planétaires (19) qui sont maintenus sur des orbites, sur lesquelles ils s'engagent par leurs rainures d'entraînement (21) prévues sur leur périphérie extérieure dans des rainures d'entraînement (8) correspondantes d'un corps creux (7) entourant les rouleaux planétaires (19), au moins les rainures d'entraînement (8) du corps creux (7) ou celles des rouleaux planétaires (19) étant agencées sous la forme de rainures hélicoïdales, afin de déplacer le corps creux (7) axialement par rapport à l'arbre d'entraînement (16) lors d'une rotation de ce dernier.

2. Dispositif de distraction selon la revendication 1, **caractérisé en ce que**,
pour l'entraînement des rouleaux planétaires (19), l'arbre d'entraînement (16) est muni d'une denture extérieure (17) de type pignon qui s'engrène dans une denture extérieure (18) prévue sur chaque rouleau planétaire (19) à côté des rainures d'entraînement (21).

3. Dispositif de distraction selon la revendication 1, **caractérisé en ce que**
pour l'entraînement des rouleaux planétaires (19), l'arbre d'entraînement (16) est relié solidairement à une cage (20) susceptible de tourner dans le corps creux (7), dans laquelle sont logés les rouleaux planétaires (19).

4. Dispositif de distraction selon l'une quelconque des revendications précédentes, **caractérisé en ce que**
le corps creux (7) entoure l'unité d'entraînement (4) de façon télescopique, et constitue une partie (2) de la broche à moelle.

5. Dispositif de distraction selon la revendication 4, **caractérisé en ce que**
l'unité d'entraînement (4) peut être fixée par rapport à un os, et **en ce que** le corps creux (7) est agencé de façon à pouvoir être dégagé.

6. Dispositif de distraction selon la revendication 4, **caractérisé en ce que**
le corps creux (7) peut être fixé sur un os, et **en ce que** l'unité d'entraînement (4) peut être dégagée avec les rouleaux planétaires (19) et une pointe (23) pouvant être sollicitée axialement, qui est positionnée coaxialement au corps creux (7).

7. Dispositif de distraction selon l'une quelconque des revendications précédentes, **caractérisé en ce que**
un joint de cardan est prévu entre l'unité d'entraînement (4) et le corps creux (7).

8. Dispositif de distraction selon l'une quelconque des revendications 1 à 3, **caractérisé en ce que**
le corps creux (7) entraîne le piston d'une pompe hydraulique au moyen de laquelle la broche à moelle peut être entraînée.

9. Dispositif de distraction selon la revendication 8, **caractérisé en ce que**
l'entraînement et la pompe hydraulique sont implantables dans le corps du patient.

10. Dispositif de distraction selon l'une quelconque des revendications précédentes, **caractérisé en ce que**
l'unité d'entraînement (4) comporte un moteur électrique (14).

11. Dispositif de distraction selon la revendication 10, **caractérisé en ce que**
le moteur électrique (14) peut être alimenté en énergie de façon inductive, notamment par un couplage à haute fréquence.

12. Dispositif de distraction selon la revendication 10, **caractérisé en ce que**

le moteur électrique (14) peut être entraîné par un accumulateur d'énergie implantable.

**13.** Dispositif de distraction selon l'une quelconque des revendications précédentes,
**caractérisé en ce que**
il est prévu un dispositif de mesure de force et/ou de déplacement pour le mouvement de la broche à moelle (1), qui est implantable dans le corps du patient.

**14.** Dispositif de distraction selon la revendication 13,
**caractérisé en ce que**
le dispositif de mesure de force fonctionne avec une jauge de contrainte.

**15.** Dispositif de distraction selon la revendication 13 ou 14,
**caractérisé en ce que**
le dispositif de mesure de déplacement fonctionne de façon potentiométrique.

**16.** Dispositif de distraction selon la revendication 13 ou 14,
**caractérisé en ce que**
un encodeur adapté au moteur sert de dispositif de mesure de déplacement.

**17.** Dispositif de distraction selon la revendication 13 ou 14,
**caractérisé en ce que**
il est prévu un capteur à effet Hall qui enregistre le nombre de rotations de l'arbre d'entraînement (16).

**18.** Dispositif de distraction selon l'une quelconque des revendications 13 à 17,
**caractérisé en ce que**
il est prévu un système de télémétrie pour la transmission sans contact des signaux de mesure émanant du corps du patient.

**19.** Dispositif de distraction selon l'une quelconque des revendications précédentes,
**caractérisé en ce que**,
dans la zone de leurs rainures d'entraînement (21), les rouleaux planétaires (19) ne sont pas en contact direct par complémentarité de formes et/ou de forces avec l'arbre d'entraînement (16).

**20.** Dispositif de distraction selon l'une quelconque des revendications précédentes,
**caractérisé en ce que**
les rouleaux planétaires (19) sont logés dans une cage rotative (20), qui est fixée axialement sur le boîtier (9) de l'arbre d'entraînement (16) en étant montée sur des paliers à roulement.

**21.** Dispositif de distraction selon l'une quelconque des revendications 1 à 18,
**caractérisé en ce que**
des rainures de palier (27) supplémentaires sont prévues dans les rouleaux planétaires (19), par lesquelles les rouleaux planétaires (19) sont fixés axialement dans des rainures de palier antagonistes (25) correspondantes, qui sont axialement immobiles.

**22.** Dispositif de distraction selon la revendication 21,
**caractérisé en ce que**
les rainures de palier (27) des rouleaux planétaires (19) s'engagent dans des rainures de palier antagonistes (25) axialement immobiles d'un corps de palier (24), qui tourne dans l'axe (I) de l'arbre d'entraînement (16).

**23.** Dispositif de distraction selon la revendication 22,
**caractérisé en ce que**
le corps de palier (24) est relié solidairement à l'arbre d'entraînement (16), et **en ce que** les rainures de palier (27, 25) des rouleaux planétaires (19) et du corps de palier (24) sont respectivement sans pas.

**24.** Dispositif de distraction selon la revendication 23,
**caractérisé en ce que**
les valeurs du diamètre moyen des rainures de palier (25) du corps de palier (24) et du diamètre du cercle primitif de la denture concordent entre le pignon d'entraînement (17) et les rouleaux planétaires (19).

**25.** Dispositif de distraction selon la revendication 22,
**caractérisé en ce que**
le corps de palier (24) est fixé axialement, mais **en ce qu'**il est monté en rotation sur l'arbre d'entraînement (16).

Fig.1

Fig.2

Fig.3

SI-SI:

Fig.4

SII-SII:

Fig.5

EP 0 959 793 B1

Fig.6

Fig.7

Fig.8

Fig.9

Fig.10

Fig.11